(19) Europäisches Patentamt
European Patent Office
Office européen des brevets

(11) Numéro de publication: **0 135 444**
**B1**

(12) **FASCICULE DE BREVET EUROPEEN**

(45) Date de publication du fascicule du brevet: **18.01.89**

(51) Int. Cl.$^4$: **C 07 D 207/28,** C 07 F 7/10, A 01 N 43/36

(21) Numéro de dépôt: **84401803.6**

(22) Date de dépôt: **12.09.84**

(54) **Dérivés pyroglutamiques, procédé de préparation, intermédiaires de synthèse de préparation de ceux-ci et compositions antibactérien ou antifongiques, cosmetiques ou pharmaceutiques.**

(30) Priorité: **12.09.83 FR 8314480**

(43) Date de publication de la demande:
**27.03.85 Bulletin 85/13**

(45) Mention de la délivrance du brevet:
**18.01.89 Bulletin 89/03**

(84) Etats contractants désignés:
**AT BE CH DE GB IT LI LU NL SE**

(56) Documents cités:
**EP-A-0 057 846**
**FR-A-2 218 342**

**CHEMICAL ABSTRACTS, vol. 80, no. 7, 18 février 1974, page 286, no. 36987t, Columbus, Ohio, US; & JP - A - 73 22 698 (TOA GOSEI CHEMICAL INDUSTRY CO., LTD.) 07-07-1973**

(73) Titulaire: **LABORATOIRES SEROBIOLOGIQUES S.A.**
**F-54 420 Pulnoy (FR)**

(72) Inventeur: **Pauly, Marc**
**10 rue Joffre**
**F-57170 Chateau Salins (FR)**

(74) Mandataire: **Portal, Gérard et al**
**Cabinet Beau de Loménie 55, rue d'Amsterdam**
**F-75008 Paris (FR)**

**EP 0 135 444 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne un nouveau dérivé pyroglutamique, une composition antibactérienne et/ou antifongique, cosmétique ou pharmaceutique les contenant, un procédé de préparation et nouveaux intermédiaires de synthèse de préparation de ceux-ci.

On connaît déjà dans la littérature le nitrile pyroglutamique encore dénommé cyano-5 pyrrolidone-2 N-non substitué ou N-substitué sans spécification d'une quelconque activité pharmacologique (voir les demandes de brevets japonaises déposées sous les n° 68/24956 le 16 avril 1968 et 69/4410 déposée le 23 janvier 1969 au nom de la Société Toa Gosei Chemical Industry Co, Ltd, publiées respectivement après examen sous les n°s 73 02538 le 25 janvier 1973 et 73 22698 le 7 juillet 1973 et dont l'abrégé a été publié respectivement dans la revue "Chemical Abstract" de 1973, *78*, 147 791 c et de 1974, *80* 36 987 t).

Dans ces deux demandes de brevets, on prépare le nitrile pyroglutamique par cyclisation d'un cyano ester avec soit $NH_3$ pour donner le nitrile pyroglutamique N-non substitué, soit par cyclisation du cyano β-formylpropionate par traitement avec une amine primaire substituée par un radical organique ayant diverses significations. On peut donc constater que les deux demandes concernent en fait le même procédé de cyclisation à partir d'un dérivé cyano à chaîne linéaire par réaction avec l'ammoniac ou une amine primaire. Cependent, ce procédé présente des inconvénients majeurs étant donné qu'il nécessite la préparation en plusieurs étapes de la chaîne linéaire et ensuite sa cyclisation par réaction avec l'ammoniac ou une amine primaire. Plusieurs étapes sont coûteuses et peu aisées à réaliser ce qui limite grandement l'application de ce procédé.

Le document EP-A-0 057 846 décrit des dérivés de pyrrolidone, ainsi qu'un procédé de synthèse utilisant une étape de silylation pour obtenir un intermédiaire 1-triméthylsilyle (voir exemple 3). Ces dérivés possèdent une activité contre l'insuffisance cérébrale.

Enfin le document FR-A-2 218 342 est relatif à des agents silylants, d'une manière générale.

La présente invention a pour but de fournir de nouveaux dérivés pyroglutamiques doués de propriétés antibactériennes ou antifongiques, en permettant ainsi de préparer des compositions antibactériennes et/ou antifongiques, cosmétiques ou pharmaceutiques.

La présente invention a encore pour but de fournir de nouveaux dérivés pyroglutamiques doués d'activités antibactériennes ou antifongiques préparés par un procédé plus aisé, permettant de partir de substances de départ simples, peu coûteuses et facilement accessibles sur le marché.

L'invention permet de résoudre ces nouveaux problèmes techniques pour la première fois d'une manière satisfaisante.

Ainsi, la présente invention fournit tout d'abord de nouveaux dérivés pyroglutamiques répondant à la formule (I) suivante:

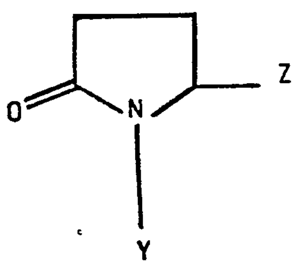

I

dans laquelle:

Y et Z ont les significations suivantes:

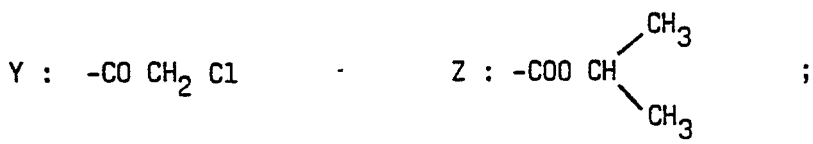

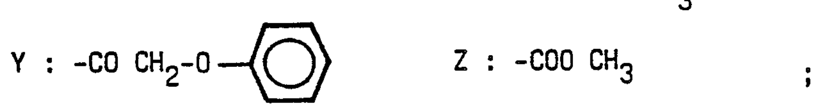

Y : $-CO\ CH_2\ Cl$ Z : $-COO\ CH_3$ ;

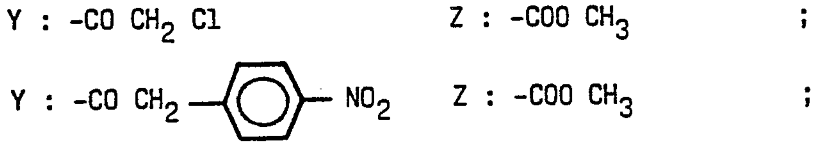

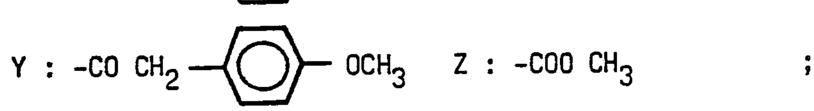

Y : 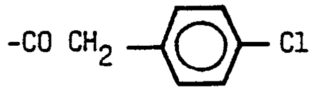 Z : $-COO\ CH_3$ ;

Y : 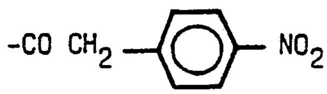 Z : $-COOH$ ;

Y : $-COCH_2-Cl$ Z : $-COOH$

L'invention concerne aussi une composition antibactérienne et/ou antifongique, caractérisée en ce qu'elle comprend, à titre d'agent antibactérien et/ou antifongique, un dérivé pyroglutamique de formule (I), tel que défini ci-dessus.

La présente invention concerne encore une composition cosmétique ou pharmaceutique, caractérisée en ce qu'elle contient, comme ingrédient actif, au moins un dérivé pyroglutamique tel que défini ci-dessus.

Cette composition comprend de 0,01 à 5% en poids d'au moins un dérivé pyroglutamique précité, de préférence de 0,1 à 5% en poids, et encore de préférence de 0,6 à 5% en poids de dérivé pyroglutamique précité.

Selon au autre aspect, la présente invention fournit un procédé de préparation d'un dérivé pyroglutamique de formule (I) tel que ci-dessus défini, caractérisé en ce qu'on fait réagir un composé de formule (II) suivante:

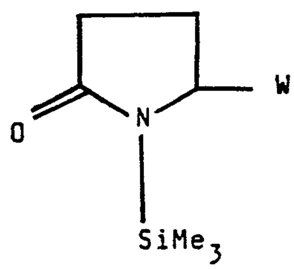

II

dans laquelle W est choisi parmi un groupe ester correspondant à Z précédemment défini ou un groupe $-COOSiMe_3$ avec un agent d'acylation, tel qu'un halogénure organique de formule Y—Hal, où Hal = halogène est en particulier du chlore, et où Y est défini comme précédemment, soit par le dicétène; et dans le cas où W est le groupe $-COOSiMe_3$, on transforme le produit intermédiaire résultant en l'acide ou l'ester correspondant par exemple par un hydrolyse ou une alcoolyse de celui-ci.

Selon une caractéristique particulière, pour préparer le composé de formule (II) précitée, dans laquelle W est un groupe ester, on fait réagir le composé de formule (IV) suivante:

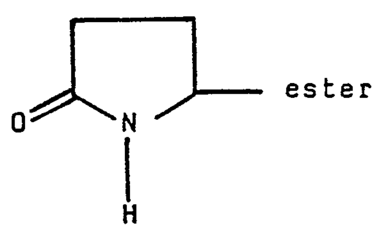

IV

avec le chlorure de triméthylsilyle.

Le composé (IV) est préparé avantageusement par estérification de l'acide pyroglutamique.

Selon une autre caractéristique du procédé de l'invention, pour préparer le composé de formule (II) précitée dans laquelle W est un group $-COOSiMe_3$, on fait réagir l'acide pyroglutamique avec le chlorure de triméthylsilyle.

Selon une autre caractéristique particulière, on fait réagir le composé de formule (II) précitée dans laquelle W est un groupe $-COOSiMe_3$ avec un chlorure organique correspondant; et le composé intermédiaire résultant est mis à réagir avec du chloroformiate d'alkyle de préférence en présence de triéthylamine pour obtenir un anhydride mixte qui est mis à réagir avec un alcool approprié pour donner l'ester désiré de formule (I).

On peut donc constater que tous les composés répondant à la formule (I) peuvent être préparés à partir de l'acide pyroglutamique qui est un produit chimique courant qui se trouve facilement dans les mélasses et notamment dans les mélasses de betterave. Il s'agit donc d'un produit peu coûteux et disponible en grande quantité.

D'autre part, l'acide et l'ester pyroglutamiques donnent aisément l'amide pyroglutamique par une réaction simple avec l'ammoniac.

D'autre part, à partir de cette substance de départ simple et non coûteuse qu'est l'acide pyroglutamique, le procédé selon l'invention prévoit l'utilisation comme réactif principal du chlorure de

triméthylsilyle ou chlorotriméthylsilane qui est un composé donnant d'excellents rendements, en combinaison avec des réactions simples de substitution avec un agent de substitution (par exemple alcoylation, acylation) tel qu'un halogènure et de préférence un chlorure organique.

La présente invention concerne également de nouveaux intermédiaires de synthèse répondant aux formules suivantes (VI) et (VIII)

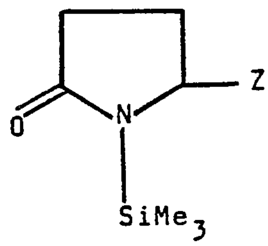

VI

dans laquelle:

Z est un groupe ester ou in groupe $-COOSiMe_3$

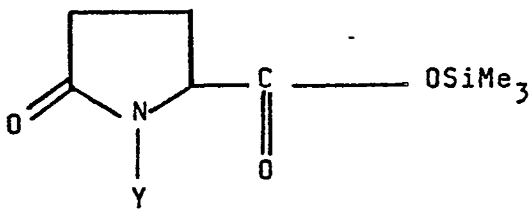

VIII

dans laquelle:

Y est tel que précédemment défini.

L'intermédiaire de synthèse répondant à la formule VI précédente est d'un intérêt primordial car le groupe triméthylsilyle substituant l'atome d'azote est aisément substitué par tous les agents de substitution connus, et en particulier les agents d'alcoylation ou d'acylation comme les halogénures d'alcoyle ou d'acyle et de préférence les chlorures d'alcoyle ou d'acyle; ou le dicétène.

La présente invention sera maintenant décrite en référence è divers exemples donnés simplement à titre d'illustration.

Exemple I

Préparation de l'intermédiaire de synthèse (VI) précité dans lequel W est un group 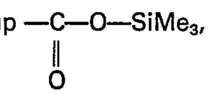, soit:

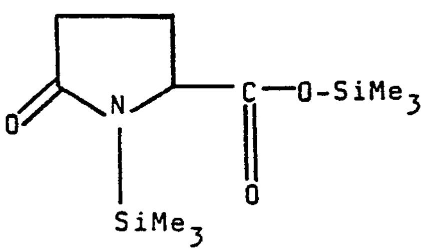

On chauffe à 60°C, jusqu'à dissolution, 200 g (1,55 M) d'acide pyroglutamique dans 650 ml (470 g, 4,65 M) de triéthylamine, puis on additionne 200 ml de toluène.

On monte la température à 65-70°C et on additonne en 1 h 30 une solution de 500 ml (423 g, 3,88 M) de chlorotriméthylsilane dans 300 ml de toluène. On garde ensuite 3 heures à reflux, on laisse refroidir, on filtre, on lave le précipité avec du toluène, on évapore et on distille.

Le rendement est de 79% en produit pur, distillant à 98°C sous 0,3 mm de mercure.

Le même produit est obtenu avec un rendement de 98% par triméthylsilylation de l'acide N-triméthylsilyl pyroglutamique lui-même préparé selon le même mode opératoire mais avec une quantité plus faible de chlorotriméthylsilane.

Exemple II (pas compris dans la portée des revendications)

Préparation de l'intermédiaire de synthèse (VIII) précité dans lequel

Y= 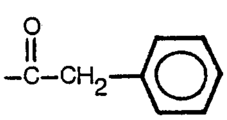, soit:

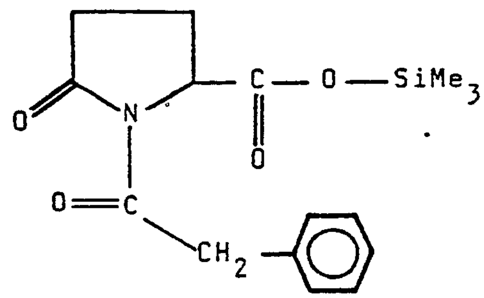

On additionne et 30 mn une solution de 13,6 g (0,088 M) de chlorure de l'acide phènyl acétique dans 25 ml de tétrahydrofuranne, à une solution de 20 g du produit obtenu dans l'exemple I dissous dans 100 ml de tétrahydrofuranne, puis on agite une nuit et évapore les solvants. Le rendement est de 85%.

Tous les autres intermédiaires de synthèse sont obtenus de la même manière par emploi d'un agent de substitution classique, comme un agent d'alcoylation ou d'acylation tel qu'un halogènure et de préférence un chlorure.

Exemple III (pas compris dans la portée des revendications)

Préparation du dérivé pyroglutamique de formule:

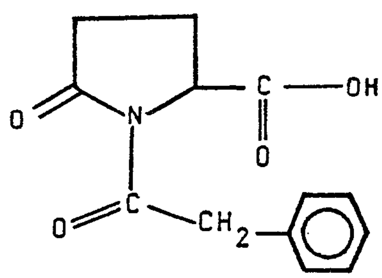

A la solution obtenue dans l'exemple II, avant évaporation, on additionne 100 ml d'eau contenant 10 ml. d'acide chlorhydrique; et on laisse en contact 15 minutes.

La phase aqueuse est extraite au chlorure de méthylène, les phases organiques sont séchées et évaporées. Le précipité est recristallisé dans le dioxane contenant un peu d'éther. Le rendement est de 78% en produit de point de fusion = 144°C.

Les autres nouveaux dérivés pyroglutamiques sont obtenus de la même manière.

Exemple IV

Préparation du nouveau dérivé pyroglutamique de formule (I) suivante:

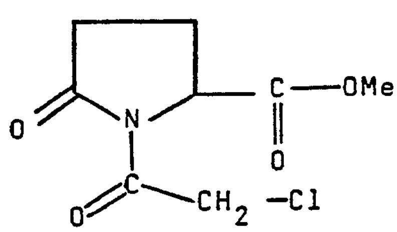

Une solution de N-chloracétyl pyroglutamate de triméthylsilyle, obtenue comme dans l'exemple II, est additionée de deux équivalents de triéthylamine. On refroidit à 0°C, puis on additionne lentement 1,2 équivalent de chloroformiate de méthyle, puis, après 15 mn, deux équivalents de méthanol. On laisse reposer 40 mn, on filtre, on évapore et on distille. Le rendement est de 85% on produit de point de fusion 66°C (éther); $E_{0,15}$ = 142°C.

Les autres composàs de formule (I) dans laquelle Z est un groupe ester et Y est un groupe acyle tel que précédemment défini sont obtenus de la même manière.

Exemple V

Préparation du nouveau dérivé de formule (I) dans laquelle

Y = 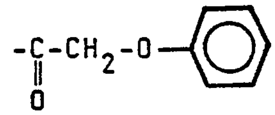

Z = 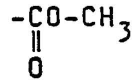 , soit

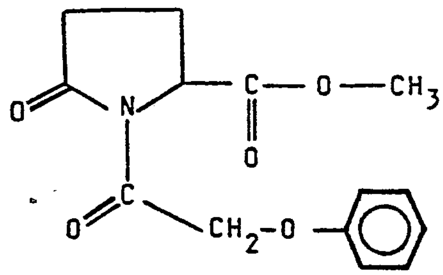

On dissout 32 g (0,15 M) de N-triméthylsilyl pyroglutamate de méthyle dans 70 ml de tétrahydrofurane, on additionne en 1 heure 25 g (0,15 M) de chlorure de phénoxyacétyle dissous dans 30 ml de tétrahydrofurane, et on porte à reflux une nuit. On obtient 25 g de produit, PF = 112°C. Les autres nouveaux composés de formule (I) dans lesquels Z est un groupe ester et Y un groupe acyle, peuvent être obtenus de la même manière.

Exemple VI

Préparation de l'intermédiaire de synthèse de formule (II) dans laquelle W est un groupe ester, par exemple —$COOC_5H_{11}$, soit:

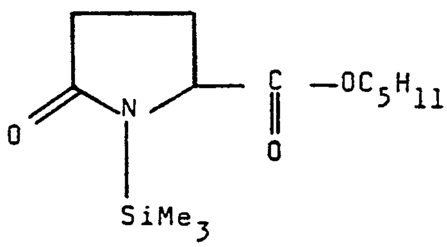

On porte à léger reflux un mélange de 100 g (0,503 M) de pyroglutamate de n-pentyle et on additionne en 30 mn, 50 ml de toluène contenant 76,5 ml (0,603 M; 65,5 g) de chlorotriméthylsilane. On garde le reflux 5 heures, on filtre, on lave le précipité au toluène et on distille. Le rendement est de 92% en produit, $E_{0,2}$ = 151—2°C. Les autres composés estérifiés de formule (II) peuvent être obtenus de la même manière.

Exemple VII

Préparation d'autres nouveaux dérivés de formule I précitée: En procédant comme décrit aux exemples III à V, on obtient par exemple les composés suivants:

TABLEAU

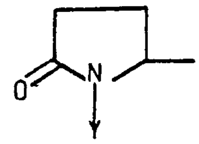

(I)

| | | | |
|---|---|---|---|
| Z | 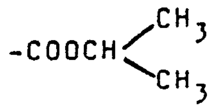 | $-COOCH_3$ | $-COOCH_3$ |
| Y | $-COCH_2Cl$ | 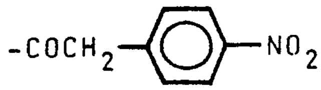 | 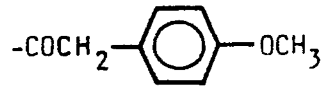 |
| Z | $-COOCH_3$ | $-COOH$ | $-COOH$ |
| Y | 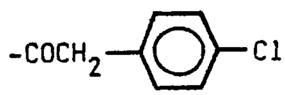 | 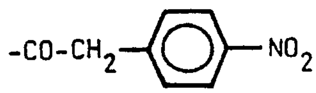 | $-COCH_2Cl$ |

Exemple XIV

Détermination de l'activité bactériostatique et fongistatique des dérivés pyroglutamiques de formule I précitée

Pour déterminer si les dérivés pyroglutamiques de formule I précitée ont un effet antibactérien ou antifongique, on procède par la méthode classique d'antibiogramme, ce qui permet de vérifier leur effet d'inhibition de croissance de divers micro-organismes, bactéries ou fonges ("Fungi").

Dans le cas où le composé testé provoque au moins l'inhibition d'un micro-organisme, d'une bactérie ou fonge dans les conditions de la méthode classique d'antibiogramme, le composé en question est considéré comme ayant un effet antibactérien s'il inhibe la crossance d'une bactérie ou antifongique s'il inhibe la croissance d'un fonge, ou le cas échéant des deux.

De préférence, l'effet d'inhibition obtenu du composé testé est comparé avec celui de substances antibactériennes ou antifongiques connues et si cet effet est inférieur ou égal à celui de la substance connue, on élimine le composé testé comme ne présentant pas d'activité antibactérienne ou antifongique suffisante.

On donne dans le tableau I ci-après des exemples de résultats obtenus avec des dérivés pyroglutamiques de formule I précitée en comparaison avec des substances connues.

Les composés ayant une activité antibactérienne ou antifongique peuvent être incorporés dans diverses compsitions à raison de 0,01% à 5% en poids par rapport au poids total de la composition, de préférence à raison de 0,1 à 5% en poids de la composition totale et encore de préférence à raison de 0,5 à 5% en poids de la composition totale.

On peut citer comme exemples de composition des produits déodorants ou capillaires grâce aux propriétés antiseptiques des produits retenus, en cosmétique, grâce aux effets bactériostatiques des produits retenus et sous forme de composition pharmaceutique en particulier dans l'utilisation contre les mycoses cutanées.

TABLEAU I

Etude de l'Activité bactériostatique et fongistatique de quelques exemples de dérivés pyroglutamique de formule (I)

| (I) 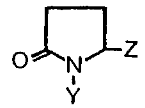 | | Diffusion en milieu de culture solide d'un échantillon de Subst. *pure* (Diamètre de la zone d'inhibition en mm) | | | | | | | | | Dilutions en Milieu de culture liquide | |
|---|---|---|---|---|---|---|---|---|---|---|---|---|
| | | Microorganisme | | | | | | | | | | |
| Y | Z | staphy | Esch. | Klebs | Prot. | Streptoc. | Alcalig. | Pseudom. | Candida. | Asperge | Bactéries | Fungi |
| $—COCH_2Cl$ | 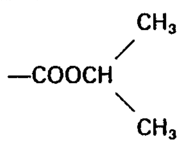 | 3,9 | 2,75 | 2,5 | 4 | 3 | 2,75 | 1 | 7 | 5,75 | Inhibition | Inhibition |
| 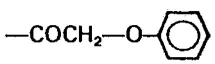 | $—COOCH_3$ | Ø | Ø | Ø | Ø | Ø | Ø | Ø | Ø | Ø | Inhibition | Croissance |
| $—COCH_2Cl$ | $—COOCH_3$ | 0,5 | 0,3 | 0,35 | 0,75 | 2,9 | 1,2 | Ø | 0,75 | 0,4 | Inhibition | Inhibition |
| 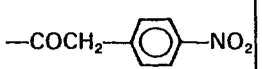 | $—COOCH_3$ | Ø | Ø | Ø | Ø | Ø | Ø | Ø | Ø | Ø | Inhibition | Inhibition |
| 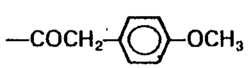 | $—COOCH_3$ | Ø | Ø | Ø | Ø | Ø | Ø | Ø | Ø | Ø | Inhibition | Croissance |
| 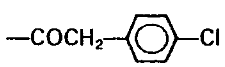 | $—COOCH_3$ | Ø | Ø | Ø | Ø | Ø | Ø | Ø | Ø | Ø | Inhibition | Croissance |
| 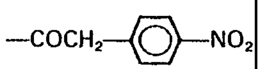 | $—COOH$ | Ø | Ø | Ø | Ø | Ø | Ø | Ø | Ø | Ø | Inhibition | Croissance |
| $—COCH_2—Cl$ | $—COOH$ | | | | | | | | | | Inhibition | Croissance |

**Revendications pour les Etats contractants: BE CH DE GB IT LI LU NL SE**

1. Dérivé pyroglutamique, caractérisé en ce qu'il répond à la formule (I) suivante:

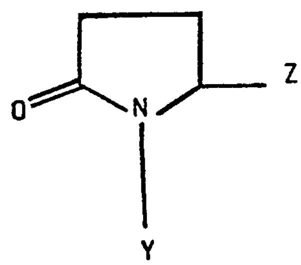

I

dans laquelle:

Y et Z ont les significations suivantes:

| | | |
|---|---|---|
| Y : $-CO\ CH_2\ Cl$ | Z : 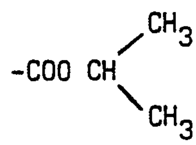 | ; |
| Y : 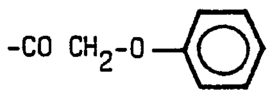 | Z : $-COO\ CH_3$ | ; |
| Y : $-CO\ CH_2\ Cl$ | Z : $-COO\ CH_3$ | ; |
| Y : 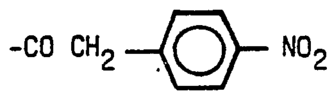 | Z : $-COO\ CH_3$ | ; |
| Y : 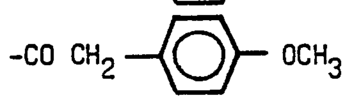 | Z : $-COO\ CH_3$ | ; |
| Y : 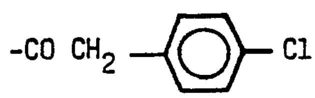 | Z : $-COO\ CH_3$ | ; |
| Y : 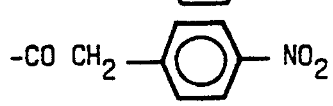 | Z : $-COOH$ | ; |
| Y : $-COCH_2-Cl$ | Z : $-COOH$ | |

2. Composition antibactérienne et/ou antifongique, caractérisée en ce qu'elle comprend, à titre d'agent antibactérien et/ou antifongique, au moins un dérivé pyroglutamique de formule (I) tel que défini à la revendication 1.

3. Composition cosmétique ou pharmaceutique, caractérisée en ce qu'elle contient, comme ingrédient actif, au moins un dérivé pyroglutamique de formule (I) tel que défini à la revendication 1.

4. Composition selon la revendication 2 ou 3, caractérisée en ce qu'elle contient de 0,01 à 5% en poids de la composition totale de dérivé pyroglutamique précité.

5. Composition selon la revendication 2 ou 4, caractérisée en ce qu'elle contient de 0,1 à 5% en poids de la composition totale de dérivé pyroglutamique précité.

6. Procédé de préparation d'un dérivé pyroglutamique de formule (I) tel que défini à la revendication 1, caractérisé en ce qu'on fait réagir un composé de formule (II) suivante:

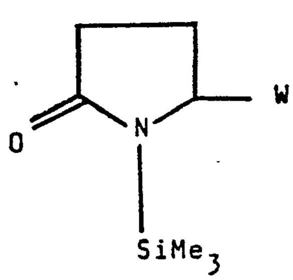

II

dans laquelle W est choisi parmi un groupe ester correspondant à Z précédemment défini ou un groupe —$COOSiMe_3$ avec un agent d'acylation, tel qu'un halogénure organique de formule Y—Hal, où Hal = halogène est en particulier du chlore, et où Y est défini comme précédemment, soit par le dicétène; et dans le cas où W est le groupe —$COOSiMe_3$, on transforme le produit intermédiaire résultant en l'acide ou l'ester correspondant par exemple par une hydrolyse ou une alcoolyse de celui-ci.

7. Procédé selon la revendication 6, caractérisé en ce que, pour préparer le composé de formule (II) précitée, dans laquelle W est un group ester, on fait réagir le composé de formule (IV) suivante:

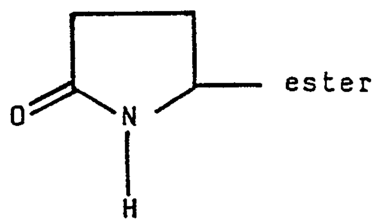

IV

avec le chlorure de triméthylsilyle.

8. Procédé selon la revendication 7, caractérisé en ce que le composé (IV) est préparé par estérification de l'acide pyroglutamique.

9. Procédé selon la revendication 6, caractérisé en ce que, pour préparer le composé de formule (II) précitée dans laquelle W est un groupe —$COOSiMe_3$, on fait réagir l'acide pyroglutamique avec le chlorure de triméthylsilyle.

10. Procédé selon la revendication 6, caractérisé en ce qu'on fait réagir le composé de formule (II) précitée dans laquelle W est un groupe —$COOSiMe_3$ avec un chlorure organique correspondant; et le composé intermédiaire résultant est mis à réagir avec du chloroformiate d'alkyle de préférence en présence de triéthylamine pour obtenir un anhydride mixte qui est mis à réagir avec un alcool approprié pour donner l'ester désiré de formule (I).

11. Intermédiaire de synthèse, caractérisé en ce qu'il répond à la formule (VI) suivante:

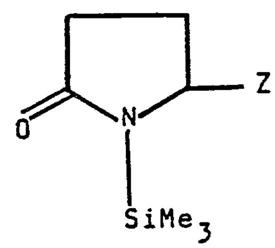

VI

dans laquelle Z est tel que défini à la revendication 1 ou un groupe —$COOSiMe_3$.

12. Intermédiaire de synthèse, caractérisé en ce qu'il répond è la formule (VIII) suivante:

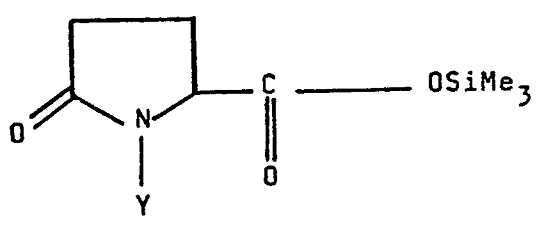

VIII

dans laquelle Y est tel que défini è la revendication 1.

**Revendications pour l'Etat contractant: AT**

1. Procédé de préparation d'un dérivé pyroglutamique de formule (I) suivante:

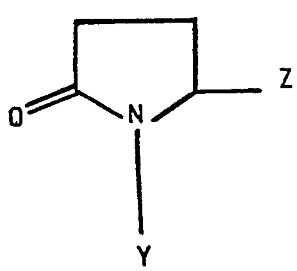

I

dans laquelle:

Y et Z ont les significations suivantes:

| | | |
|---|---|---|
| Y : $-CO\ CH_2\ Cl$ | Z : 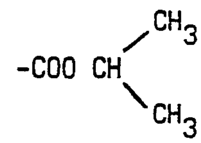 | ; |
| 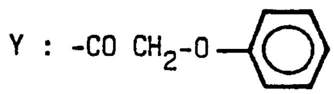 | Z : $-COO\ CH_3$ | ; |
| Y : $-CO\ CH_2\ Cl$ | Z : $-COO\ CH_3$ | ; |
| 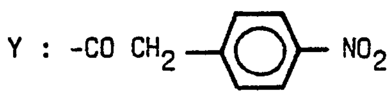 | Z : $-COO\ CH_3$ | ; |
| 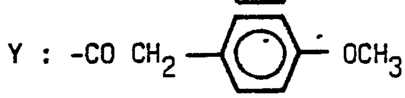 | Z : $-COO\ CH_3$ | ; |
| 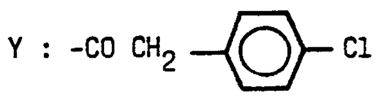 | Z : $-COO\ CH_3$ | ; |
| 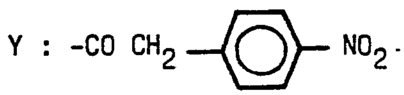 | Z : $-COOH$ | ; |
| Y : $-COCH_2-Cl$ | Z : $-COOH$ | |

caractérisé en ce qu'on fait réagir un composé de formule (II) suivante:

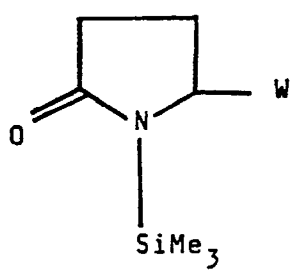

II

dans laquelle W est choisi parmi un groupe ester correspondant à Z ci-dessus défini, ou un groupe $—COOSiMe_3$;
avec un agent d'acylation, tel qu'un halogénure organique de formule Y-Hal, où Hal = halogène en particulier du chlore, et où Y est défini comme précédemment, soit par le dicétène;
et dans le cas où W est le groupe $-COOSiMe_3$, on transforme le produit intermédiaire résultant en acide ou l'ester correspondant par exemple par une hydrolyse ou une alcoolyse de celui-ci.

2. Procédé selon la revendication 1, caractérisé en ce que, pour préparer le composé de formule (II) précitée, dans laquelle W est un groupe ester, on fait réagir le composé de formule (IV) suivante:

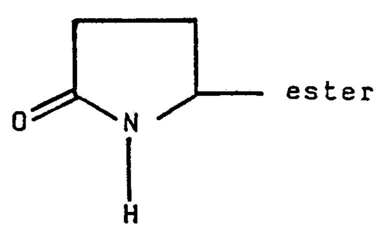

IV

avec le chlorure de triméthylsilyle.

3. Procédé selon la revendication 2, caractérisé en ce que le composé (IV) est préparé par estérification de l'acide pyroglutamique.

4. Procédé selon la revendication 1, caractérisé en ce que, pour préparer le composé de formule (II) précitée dans laquelle W est un group $—COOSiMe_3$, on fait réagir l'acide pyroglutamique avec le chlorure de triméthylsilyle.

5. Procédé selon la revendication 1, caractérisé en ce qu'on fait réagir le composé de formule (II) précitée dans laquelle W est un groupe —$COOSiMe_3$ avec un chlorure organique correspondant; et le composé intermédiaire résultant est mis à réagir avec du chloroformiate d'alkyle de préférence en présence de triéthylamine pour obtenir un anhydride mixte qui est mis à réagir avec un alcool approprié pour donner l'ester désiré de formule (I).

6. Procédé ce préparation d'une composition antibactérienne et/ou antifongique, caractérisé en ce qu'on incorpore, à titre d'agent antibactérien et/ou antifongique, au moins un dérivé pyroglutamique de formule (I) tel que défini à la revendication 1.

7. Procédé de préparation d'une composition cosmétique ou pharmaceutique, caractérisé en ce qu'on incorpore, comme ingrédient actif, au moins un dérivé pyroglutamique de formule (I) tel que défini à la revendication 1.

8. Procédé selon la revendication 6 ou 7, caractérisé en ce qu'on incorpore de 0,01 à 5% en poids du dérivé pyroglutamique précité.

9. Procédé selon la revendication 8, caractérisé en ce qu'on incorpore de 0,1 à 5% en poids du dérivé pyroglutamique précité.

**Patentansprüche für die Vertragsstaaten: BE CH DE FR GB IT LI LU NL SE**

1. Pyroglutaminsäure-Derivat der folgenden Formel (I)

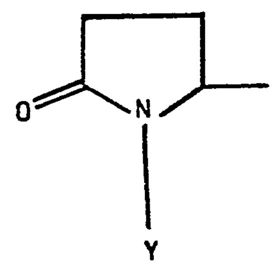

I,

in der Y und Z folgende Bedeutungen haben:

Y : $-CO\ CH_2\ Cl$ Z : 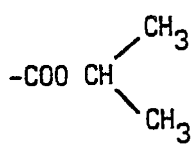 ;

Y : 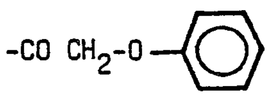 Z : $-COO\ CH_3$ ;

Y : $-CO\ CH_2\ Cl$ Z : $-COO\ CH_3$ ;

Y : 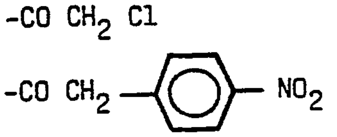 Z : $-COO\ CH_3$ ;

Y : 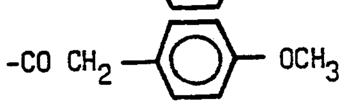 Z : $-COO\ CH_3$ ;

Y : 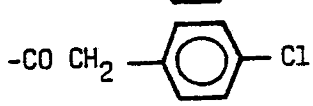 Z : $-COO\ CH_3$ ;

Y : 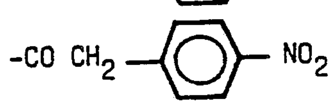 Z : $-COOH$ ;

Y : $-COCH_2-Cl$ Z : $-COOH$

2. Antibakterielle und/oder fungizide Zusammensetzung, dadurch gekennzeichnet, daß sie als antibakterielles und/oder fungizides Mittel mindestens ein Pyroglutaminsäure-Derivat der Formel (I) nach Anspruch 1 enthält.

3. Kosmetische oder pharmazeutische Zusammensetzung, dadurch gekennzeichnet, daß sie als Wirkstoff mindestens ein Pyroglutaminsäure-Derivat der Formel (I) nach Anspruch 1 enthält.

4. Zusammensetzung nach Anspruch 2 oder 3, dadurch gekennzeichnet, daß sie von 0,01 bis 5 Gew.-%, bezogen auf die Gesamtzusammensetzung, des Pyroglutaminsäure-Derivats enthält.

5. Zusammensetzung nach Anspruch 2 oder 4, dadurch gekennzeichnet, daß sie von 0,1 bis 5 Gew.-%, bezogen auf die Gesamtzusammensetzung, des Pyroglutaminsäure-Derivats enthält.

6. Verfahren zur Herstellung eines Pyroglutaminsäure-Derivats der Formel (I) nach Anspruch 1, dadurch gekennzeichnet, daß eine Verbindung der folgenden Formel (II)

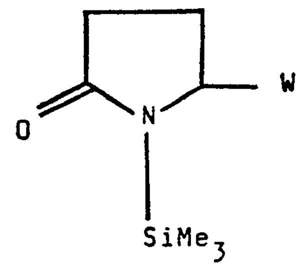

II,

in der W unter einer dem oben angegebenen Z entsprechenden Estergruppe gewählt wird oder —$COOSiMe_3$ ist, mit einem Acylierungsmittel, wie einem organischen Halogenid der Formel Y—Hal, in der Hal Halogen and insbesondere Chlor ist und Y wie oben definiert oder Diketen ist, umgesetzt wird, und im Fall, daß W —$COOSiMe_3$ ist, das entstandene Zwischenprodukt in die entsprechende Säure oder den entsprechenden Ester beispielsweise durch Hydrolyse oder Alkoholyse umgewandelt wird.

7. Verfahren nach Anspruch 6, dadurch gekennzeichnet, daß zur Herstellung der Verbingdung der Formel (II), in der W eine Estergruppe ist, die Verbindung der folgenden Formel (IV)

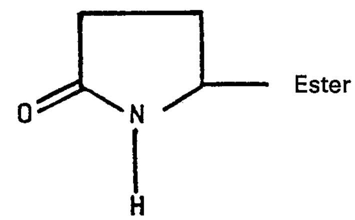

IV

mit Trimethylsilylchlorid umgesetzt wird.

8. Verfahren nach Anspruch 7, dadurch gekennzeichnet, daß die Verbindung der Formel (IV) durch Veresterung der Pyroglutaminsäure hergestellt wird.

9. Verfahren nach Anspruch 6, dadurch gekennzeichnet, daß zur Herstellung der Verbindung der Formel (II), in der W —$COOSiMe_3$ ist, die Pyroglutaminsäure mit dem Trimethylsilylchlorid umgesetzt wird.

10. Verfahren nach Anspruch 6, dadurch gekennzeichnet, daß die Verbindung der Formel (II), in der W —$COOSiMe_3$ ist, mit dem entsprechenden organischen Chlorid umgesetzt wird, die entstandene Zwischenverbindung mit einem Alkylchlorformiat vorzugsweise in Gegenwart von Triethylamin zu einem gemischten Anhydrid umgesetzt wird, das mit einem entsprechenden Alkohol zum gewünschten Ester der Formel (I) umgewandelt wird.

11. Zwischenprodukt der folgenden Formel (VI)

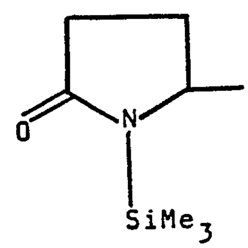

VI,

in der Z nach Anspruch 1 definiert oder —$COOSiMe_3$ ist.

12. Zwischenprodukt der folgenden Formel (VIII)

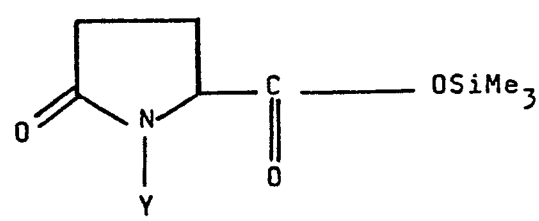

VIII,

in der Y nach Anspruch 1 definiert ist.

**Patentansprüche für den Vertragsstaat: AT**

1. Verfahren zur Herstellung eines Pyroglutaminsäure-Derivats der folgenden Formel (I)

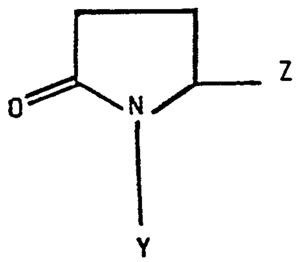

I,

in der Y und Z folgende Bedeutungen haben:

| | | |
|---|---|---|
| Y : -CO $CH_2$ Cl | Z : -COO 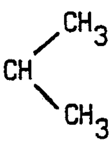 | ; |
| Y : -CO $CH_2$-O- 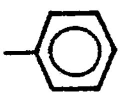 | Z : -COO $CH_3$ | ; |
| Y : -CO $CH_2$ Cl | Z : -COO $CH_3$ | ; |
| Y : -CO $CH_2$ 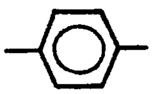 $NO_2$ | Z : -COO $CH_3$ | ; |
| Y : -CO $CH_2$ 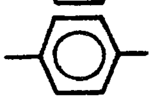 $OCH_3$ | Z : -COO $CH_3$ | ; |
| Y : -CO $CH_2$ 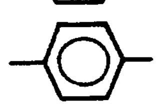 Cl | Z : -COO $CH_3$ | ; |
| Y : -CO $CH_2$ 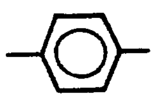 $NO_2$ | Z : -COOH | ; |
| Y : -$COCH_2$-Cl | Z : -COOH | |

dadurch gekennzeichnet, daß eine Verbindung der folgenden Formel (II)

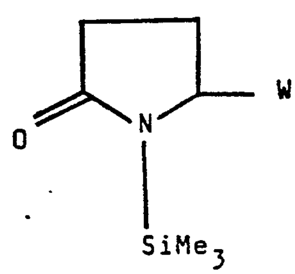

II,

in der W unter einer dem oben angegebenen Z entsprechenden Estergruppe gewählt wird oder —$COOSiMe_3$ ist, mit einem Acylierungsmittel, wie einem organischen Halogenid der Formel Y—Hal, in der Hal Halogen und insbesondere Chlor und Y wie oben definiert oder Diketen ist, umgesetzt wird, und im Fall, daß W —$COOSiMe_3$ ist, das entstandene Zwischenprodukt in die entsprechende Säure oder den entsprechenden Ester beispielsweise durch Hydrolyse oder Alkoholyse umgewandelt wird.

2. Verfahren nach Anspruch 1, dadurch gekennzeichnet, daß zur Herstellung der Verbindung der Formel (II), in der W ein Ester ist, die Verbindung der folgenden Formel (IV)

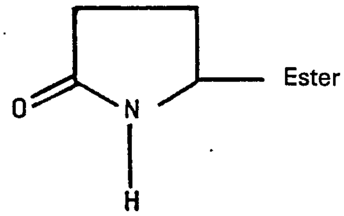

IV

mit Trimethylsilylchlorid umgesetzt wird.

3. Verfahren nach Anspruch 2, dadurch gekennzeichnet, daß die Verbindung der Formel (IV) durch Veresterung von Pyroglutaminsäure hergestellt wird.

4. Verfahren nach Anspruch 1, dadurch gekennzeichnet, daß zur Herstellung der Verbindung der Formel (II), in der W —$COOSiMe_3$ ist, Pyroglutaminsäure mit dem Trimethylsilylchlorid umgesetzt wird.

5. Verfahren nach Anspruch 1, dadurch gekennzeichnet, daß die Verbindung der Formel (II), in der W —$COOSiMe_3$ ist, mit dem entsprechenden organischen Chlorid umgesetzt wird, die entstandene Zwischenverbindung mit einem Alkylchlorformiat vorzugsweise in Gegenwart von Triethylamin zu einem gemischten Anhydrid umgesetzt wird, das mit einem entsprechenden Alkohol zum gewünschten Ester der Formel (I) umgewandelt wird.

6. Verfahren zur Herstellung einer antibakteriellen und/oder fungiziden Zusammensetzung, dadurch gekennzeichnet, daß als antibakterielles und/oder fungizides Mittel mindestens ein Pyroglutaminsäure-Derivat der Formel (I) nach Anspruch 1 verwendet wird.

7. Verfahren zur Herstellung einer kosmetischen oder pharmazeutischen Zusammensetzung, dadurch gekennzeichnet, daß als Wirkstoff mindestens ein Pyroglutaminsäure-Derivat der Formel (I) nach Anspruch 1 verwendet wird.

8. Verfahren nach Anspruch 6 oder 7, dadurch gekennzeichnet, daß von 0,01 bis 5 Gew.-% des Pyroglutaminsäure-Derivats verwendet werden.

9. Verfahren nach Anspruch 8, dadurch gekennzeichnet, daß von 0,1 bis 5 Gew.-% des Pyroglutaminsäure-Derivats verwendet werden.

**Claims for the Contracting States: BE CH DE GB IT LI LU NL SE**

1. Pyroglutamic derivative, characterized in that it corresponds to the following formula (I):

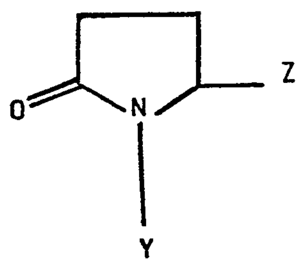

I

in which:
Y and Z have the following meanings:

Y : $-CO\ CH_2\ Cl$ Z : $-COO$ 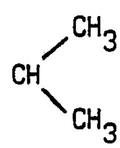 ;

Y : $-CO\ CH_2-O-$ 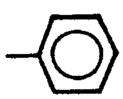 Z : $-COO\ CH_3$ ;

Y : $-CO\ CH_2\ Cl$ Z : $-COO\ CH_3$ ;

Y : $-CO\ CH_2$ 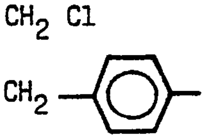 $NO_2$ Z : $-COO\ CH_3$ ;

Y : $-CO\ CH_2$ 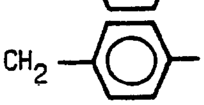 $OCH_3$ Z : $-COO\ CH_3$ ;

Y : $-CO\ CH_2$ 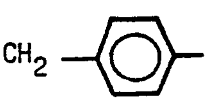 Cl Z : $-COO\ CH_3$ ;

Y : $-CO\ CH_2-$ 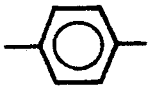 $-NO_2$ Z : $-COOH$ ;

Y : $-COCH_2-Cl$ Z : $-COOH$

2. Antibacterial and/or antifungal composition, characterized in that it comprises, by way of an antibacterial and/or antifungal agent, at least one pyroglutamic derivative of formula (I) as defined in Claim 1.

3. Cosmetic or pharmaceutical composition, characterized in that it contains, as active ingredient, at least one pyroglutamic derivative of formula (I) as defined in Claim 1.

4. Composition according to Claim 2 or 3, characterized in that it contains from 0.01 to 5% by weight of the total composition of abovementioned pyroglutamic derivative.

5. Composition according to Claim 2 or 4, characterized in that it contains from 0.1 to 5% by weight of the total composition of abovementioned pyroglutamic derivative.

6. Process for preparing a pyroglutamic derivative of formula (I) as defined in Claim 1, characterized in that a compound of the following formula (II):

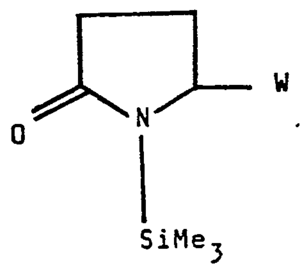

II

in which W is chosen from an ester group corresponding to Z defined above or a $-COOSiMe_3$ group, is reacted with an acylating agent such as an organic halide of formula Y—Hal, where Hal = halogen is, in particular, chlorine and where Y is defined as above, or with diketene; and, in the case where W is a $-COOSiMe_3$ group, the resulting intermediate product is converted to the corresponding acid or ester, for example by a hydrolysis or alcoholysis of this product.

7. Process according to Claim 6, characterized in that, to prepare the compound of the abovementioned formula (II) in which W is an ester group, the compound of the following formula (IV):

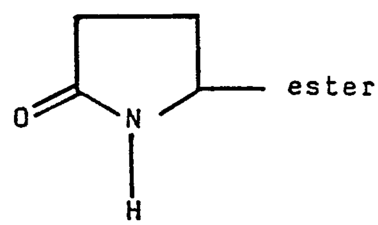

IV

is reacted with trimethylsily chloride.

8. Process according to Claim 7, characterized in that the compound (IV) is prepared by the esterification of pyroglutamic acid.

9. Process according to Claim 6, characterized in that, to prepare the compound of the abovementioned formula (II) in which W is a $-COOSiMe_3$ group, pyroglutamic acid is reacted with trimethylsilyl chloride.

10. Process according to Claim 6, characterized in that the compound of the abovementioned formula (II) in which W is a $-COOSiMe_3$ group is reacted with a corresponding organic chloride; and the resulting intermediate compound is reacted with alkyl chloroformate, preferably in the presence of triethylamine, to obtain a mixed anhydride which is reacted with a suitable alcohol to give the desired ester of formula (I).

11. Synthesis intermediate, characterized in that it corresponds to the following formula (VI):

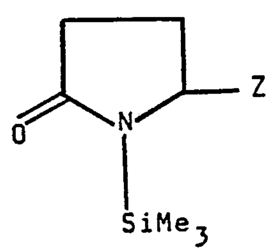

VI

in which Z is as defined in Claim 1 or a $-COOSiMe_3$ group.

12. Synthesis intermediate, characterized in that it corresponds to the following formula (VIII):

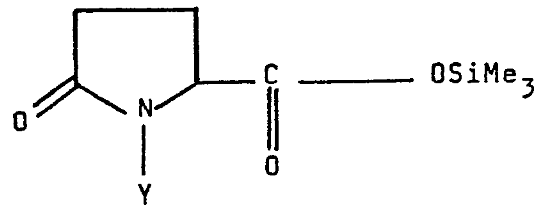

VIII

in which Y is as defined in Claim 1.

**Claims for the Contracting State: AT**

1. Process for preparing a pyroglutamic derivative of the following formula (I):

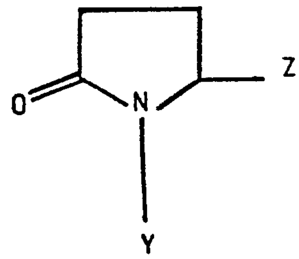

I

in which:
Y and Z have the following meanings:

Y : -CO $CH_2$ Cl    Z : -COO 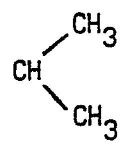 ;

Y : -CO $CH_2$-O- 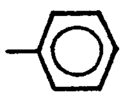    Z : -COO $CH_3$ ;

Y : -CO $CH_2$ Cl    Z : -COO $CH_3$ ;

Y : -CO $CH_2$ 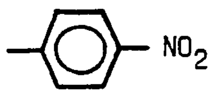    Z : -COO $CH_3$ ;

Y : -CO $CH_2$ 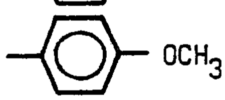    Z : -COO $CH_3$ ;

Y : -CO $CH_2$ 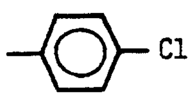    Z : -COO $CH_3$ ;

Y : -CO $CH_2$ 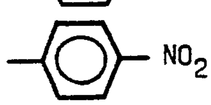    Z : -COOH ;

Y : -$COCH_2$-Cl    Z : -COOH

characterized in that a compound of the following formula (II):

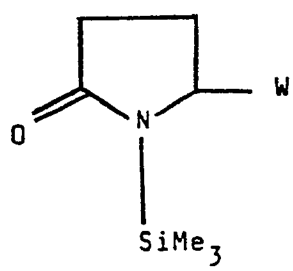

II

in which W is chosen from an ester group corresponding to Z defined above, or —$COOSiMe_3$ group; is reacted with an acylating agent such as an organic halide of formula Y—Hal, where Hal = halogen, especially chlorine, and where Y is defined as above, or with diketene; and, in the case where W is a —$COOSiMe_3$ group, the resulting intermediate product is converted to the corresponding acid or ester, for example by a hydrolysis or alcoholysis of this product.

2. Process according to Claim 1, characterized in that, to prepare the compound of the abovementioned formula (II) in which W is an ester group, the compound of the following formula (IV):

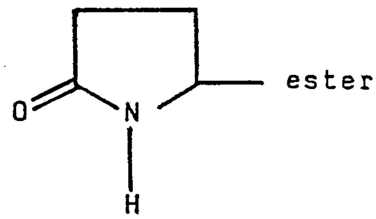

IV

is reacted with trimethysilyl chloride.

3. Process according to Claim 2, characterized in that the compound (IV) is prepared by the esterification of pyroglutamic acid.

4. Process according to Claim 1, characterized in that, to prepare the compound of the abovementioned formula (II) in which W is a —$COOSiMe_3$ group, pyroglutamic acid is reacted with trimethylsilyl chloride.

5. Process according to Claim 1, characterized in that the compound of the abovementioned formula (II) in which W is a —$COOSiMe_3$ group is reacted with a corresponding organic chloride; and the resulting intermediate compound is reacted with alkyl chloroformate, preferably in the presence of triethylamine, to obtain a mixed anhydride which is reacted with a suitable alcohol to give the desired ester of formula (I).

6. Process for preparing an antibacterial and/or antifungal composition, characterized in that at least one pyroglutamic derivative of formula (I) as defined in Claim 1 is incorporated by way of an antibacterial and/or antifungal agent.

7. Process for preparing a cosmetic or pharmaceutical composition, characterized in that at least one pyroglutamic derivative of formula (I) as defined in Claim 1 is incorporated as active ingredient.

8. Process according to Claim 6 or 7, characterized in that from 0.01 to 5% by weight of the abovementioned pyroglutamic derivative is incorporated.

9. Process according to Claim 8, characterized in that from 0.1 to 5% by weight of the abovementioned pyroglutamic derivative is incorporated.